(19) Europäisches Patentamt
European Patent Office
Office européen des brevets

(11) Veröffentlichungsnummer: **0 007 574**
**B1**

(12) # EUROPÄISCHE PATENTSCHRIFT

(45) Veröffentlichungstag der Patentschrift: **25.05.83**

(51) Int. Cl.³: **C 09 K 3/34,** C 07 C 69/92, C 07 C 121/52, C 07 C 121/75, C 07 C 69/54, C 07 C 69/76

(21) Anmeldenummer: **79102531.5**

(22) Anmeldetag: **18.07.79**

(54) **Flüssig-kristalline Polymerphase mit cholesterischer Struktur, Verfahren zu ihrer Herstellung und ihre Verwendung.**

(30) Priorität: **20.07.78 DE 2831909**

(43) Veröffentlichungstag der Anmeldung: **06.02.80 Patentblatt 80/3**

(45) Bekanntmachung des Hinweises auf die Patenterteilung: **25.05.83 Patentblatt 83/21**

(84) Benannte Vertragsstaaten: **AT BE CH DE FR GB IT LU NL SE**

(56) Entgegenhaltungen: **DE-A-2 722 589**

(73) Patentinhaber: **BASF Aktiengesellschaft, Carl-Bosch-Strasse 38, D-6700 Ludwigshafen (DE)**

(72) Erfinder: **Portugall, Michael, Dipl.-Chem., Steinheimer Strasse 53, D-6228 Eltville (DE)**
Erfinder: **Finkelmann, Heino, Dr. Dipl.-Chem., Goslarer Strasse 59, D-3392 Clausthal-Zellerfeld (DE)**
Erfinder: **Ringsdorf, Helmut, Dr. Dipl.-Chem., Kehlweg 51, D-6500 Mainz-Gonsenheim (DE)**

EP 0 007 574 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Flüssig-kristalline Polymerphase mit cholesterischer Struktur, Verfahren zu ihrer Herstellung und ihre Verwendung

Die Erfindung bezieht sich auf eine flüssig-kristalline Polymerphase mit cholesterischer Struktur, die aus chiralen und nematischen Komponenten besteht, sowie auf Verfahren zu ihrer Herstellung und ihre Verwendung.

Bisher sind niedermolekulare Flüssigkristalle mit nematischer, smektischer oder cholesterischer Struktur bekannt, die auf Grund ihrer optischen Eigenschaften u. a. Anwendungen in der Optoelektronik und zerstörungsfreien Materialprüfung gefunden haben. Die niedermolekularen cholesterischen Flüssigkristalle sind wegen ihrer hohen optischen Rotation und des Circulardichroismus, der durch selektive Reflexion von circularpolarisiertem Licht der Wellenlänge $\lambda_R$ zustande kommt, von besonderer technischer Bedeutung. Ihre helicale Überstruktur, charakterisiert durch die Ganghöhe p, steht dabei im direkten Zusammenhang mit der Wellenlänge des reflektierten Lichtes $\lambda_R$.

Es wurde auch beschrieben, daß man in niedermolekularen nematischen Flüssigkristallen durch Zumischen von chiralen Verbindungen cholesterische Helixstrukturen induzieren kann (Z. Naturforschung, 28A, 799, 1973), wobei die Ganghöhe p und die Wellenlänge $\lambda_R$ von der Konstitution und Konzentration der chiralen Komponente abhängt und dementsprechend variiert werden kann. Nachteiligerweise beobachtet man dieses optische Verhalten der niedermolekularen cholesterischen Verbindungen bzw. der niedermolekularen induziert cholesterischen Gemische nur in der flüssig-kristallinen Phase und damit in einem definierten Temperaturbereich, ohne daß die Möglichkeit besteht, die cholesterische Struktur in einer festen Phase zu erhalten.

Es ist zwar ein Copolymerisat bekannt, dessen cholesterische Struktur auch in der festen Phase erhalten bleibt (Makromol. Chem. 179, 829–832, 1978). Die cholesterische Struktur wird jedoch nur bei einem Monomerenverhältnis von etwa 1 : 1 beobachtet; eine Variation der Ganghöhe p und der Wellenlänge $\lambda_R$ ist nicht möglich.

Es ist somit Aufgabe der vorliegenden Erfindung, eine flüssig-kristalline Polymerphase mit cholesterischer Struktur vorzuschlagen, die die aufgezeigten Nachteile nicht aufweist, sondern vielmehr eine Variation der Wellenlänge des reflektierten Lichtes $\lambda_R$ gestattet und deren cholesterische Struktur ohne wesentliche Veränderung in den Glaszustand des Polymeren überführbar ist und im festen Zustand erhalten bleibt.

Überraschenderweise wird diese Aufgabe gelöst durch eine flüssig-kristalline Polymerphase mit cholesterischer Struktur, bestehend aus mindestens einer Komponente, welche ohne Zusatz einer weiteren Komponente eine nematische Struktur aufweist, und mindestens einer weiteren Komponente mit chiraler Struktur.

Die Polymerphase ist dadurch gekennzeichnet, daß sie ein nematogenes Homo- oder Copolymer mit Einheiten der folgenden Formel enthält:

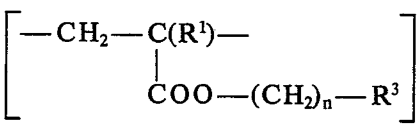

in der

$R^1$ Wasserstoff oder eine Methylgruppe ist,
n eine ganze Zahl von 1 bis 6 ist,
$R^3$ der

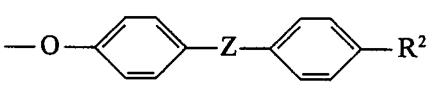
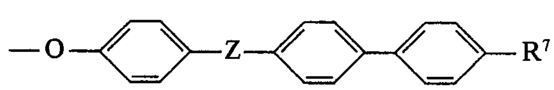

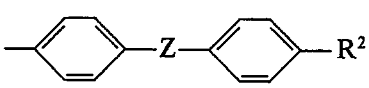
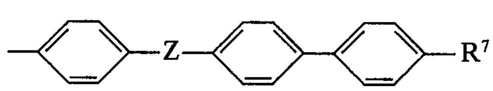

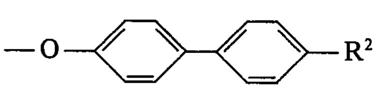

oder

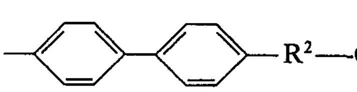 Gruppe

entspricht, wobei

Z eine COO- oder OCO-Gruppe ist und
$R^2$ eine $-O(CH_2)_mH$-, $-(CH_2)_mH$-, oder $-COO-(CH_2)_mH$-Gruppe ist, in der m eine ganze Zahl von 1 bis 6 ist, und
$R^7$ Wasserstoff oder $R^2$ ist.

Die Aufgabe wird ferner gelöst durch das in den Ansprüchen genannte Verfahren und die Verwendung.

Unter anderem bevorzugt sind solche Homo- oder Copolymerisate mit nematogenen Einheiten der oben angegebenen Formel, in denen der Rest $R^3$ einer

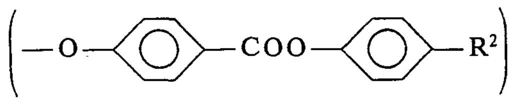 -Gruppe

oder einer

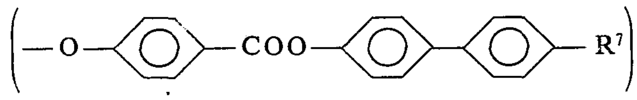 -Gruppe

entspricht.

Nematische Homopolymere aus Einheiten des genannten Typs sind z. T. in Makromol. Chem. 179, 273 – 276 (1978) veröffentlicht. Sie zeigen insbesondere dann eine nematische Struktur, wenn n oder m klein ist und somit mindestens eine der C-Ketten kurz ist.

In der erfindungsgemäßen Polymerphase mit induziert cholesterischer Struktur ist das nematische Homopolymere mit einem oder mehreren niedermolekularen oder polymeren chiralen Stoffen gemischt; vorzugsweise ist die erfindungsgemäße flüssig-kristalline Polymerphase ein Copolymer mit mindestens einer Komponente der oben genannten Formel und mindestens einer weiteren polymerisierbaren chiralen Komponente.

Die niedermolekulare chirale Komponente muß nicht notwendigerweise mesomorph sein, ist jedoch besonders vorteilhaft eine cholesterische Verbindung wie z. B. p-Methoxybenzyliden-p′-aminozimtsäure-akt-octylester oder p-Cyanobenzyliden-1-(−)-α-methylbenzylamin. Diese niedermolekularen chiralen Komponenten sind in ihrer Molekülstruktur ähnlich aufgebaut wie das Monomere des nematischen Homopolymers, was zu einer verbesserten Mischbarkeit der beiden Komponenten führt. Außerdem sind aufgrund der flüssig-kristallinen Struktur dieser niedermolekularen chiralen Komponenten und der damit verbundenen relativ hohen »helical twisting power« Reflexionswellenlängen bis zum sichtbaren Bereich einstellbar.

Vorzugsweise besteht die chirale Komponente zur Induzierung der cholesterischen Struktur aus einem Polymer (insbesondere aus einem Copolymer mit nematischen Comonomeren) mit Einheiten der folgenden Formel, welche große Ähnlichkeit zu der Formel der nematischen Komponente zeigt:

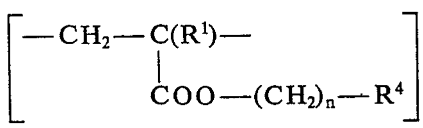

Es ist

$R^1$ Wasserstoff oder eine Methylgruppe
n eine ganze Zahl von 1 bis 10
$R^4$ eine

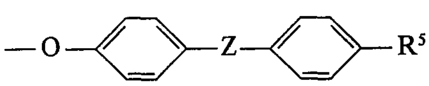

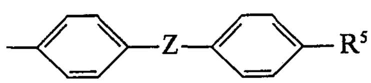

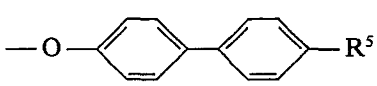

oder

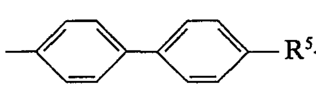 -Gruppe

wobei

Z eine COO- oder OCO-Gruppe ist und
$R^5$ $-CH=N-R^6$, $-OR^6$, $-COOR^6$, $-CH=CH-COOR^6$ oder $-R^6$ ist, wobei
$R^6$ eine Alkylgruppe mit 4 bis 10 C-Atomen und mindestens einem asymmetrischen C-Atom, oder dem $-CH(CH_3)-C_6H_5$-Rest bedeutet.

Vorzugsweise ist $R^6$ eine verzweigte Alkylgruppe, z. B. Amyl- oder Octylgruppe oder zeigt beispielsweise eine der folgenden Strukturen:

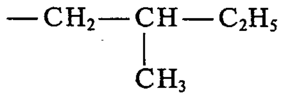 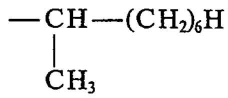 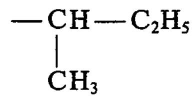

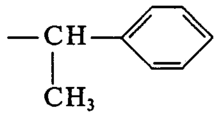

Besonders bevorzugt sind solche chiralen Komponenten, in denen $R^4$ eine

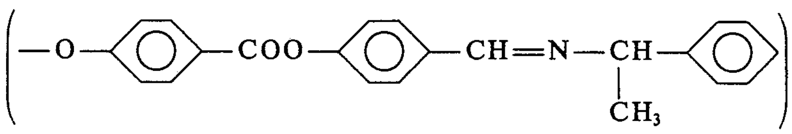 -Gruppe

oder eine

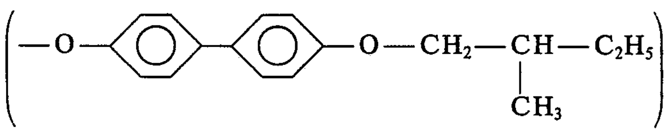 -Gruppe

darstellt.

Die chirale Komponente kann als Monomer oder Polymer ebenfalls flüssig-kristalline Struktur aufweisen, doch ist dies zur Lösung der gestellten Aufgabe nicht unbedingt erforderlich.

Die chirale Komponente wird auch als Homopolymer im Gemisch mit dem nematischen Homopolymer eingesetzt. Ganz besonders vorteilhaft ist die erfindungsgemäße Polymerphase jedoch ein Copolymer, das aus Monomeren mit den Formeln:

$$CH_2=C(R^1)-COO-(CH_2)_n-R^3$$

und

$$CH_2=C(R^1)-COO-(CH_2)_n-R^4$$

hergestellt wurde.

Die zuerst genannte Formel beschreibt die nematische Komponente, die zweite Formel die chirale Komponente. $R^1$, $R^3$, $R^4$ und n haben die obengenannte Bedeutung.

Die Copolymerisation des nematogenen Monomeren mit dem chiralen Monomeren führt, ausgehend von einer Monomerenmischung mit der Konzentration $x_1$, nur dann zu einem Copolymerisat mit dem Einbauverhältnis entsprechend der Monomerenkonzentration $x_1$, wenn die Copolymerisationsparameter der Monomerenkomponenten vergleichbarer Größenordnung sind. Das ist besonders dann von Bedeutung, wenn problemlos, z. B. ohne Berücksichtigung der Reaktionskinetik, ein cholesterisches Copolymer bestimmter Zusammensetzung hergestellt werden soll. Deshalb wählt man vorzugsweise Monomerkomponenten, die vergleichbare Copolymerisationsparameter aufweisen, nämlich Acrylsäure- oder Methacrylsäurealkylester, die sich in erster Linie durch den Substituenten $R^3$ bzw. $R^4$ in der $\omega$-Stellung der Alkylkette unterscheiden.

Bei Copolymeren von nematogenen Komponenten mit chiralen, aber selbst nicht mesomorphen Komponenten kann die chirale Komponente nur bis zu einer bestimmten Grenzkonzentration zur nematischen Phase zugegeben werden, da sonst die induziert cholesterische Phase zerstört wird. Die Grenzkonzentration wird dabei im wesentlichen von der Molekülstruktur der chiralen Komponente bestimmt. Um zu hohen Konzentrationen der chiralen Komponente zu gelangen (größer als etwa 10 Mol.%), entspricht diese in ihrer Molekülstruktur vorteilhafterweise weitgehend der Molekülstruktur der nematischen Komponente.

Aus diesem Grund besteht das bevorzugte Copolymer insbesondere aus nematischen und chiralen Komponenten der obengenannten Formeln, in denen auch $R^3$ und $R^4$ vorzugsweise weitgehend ähnlich sind und sich insbesondere nur durch die $R^2$-Gruppe und die $(-CH=N-R^6)$-, $(OR^6)$-, $(-COOR^6)$-,

$(-CH=CH-COOR^6)$- oder $(-R^6)$-Gruppe unterscheiden.

Die bevorzugten chiralen Monomere zur Herstellung des induziert cholesterischen Copolymers unterscheiden sich somit im wesentlichen nur durch das chirale Strukturelement von den nematogenen Monomeren; die übrigen Strukturelemente, wie z. B. die polymerisationsfähige Gruppe, sind vorzugsweise identisch, da auf diese Weise vergleichbare Copolymerisationsparameter auftreten. Mit der Wahl des chiralen Strukturelementes wird die »helical twisting power« und damit die Größe der reflektierten Wellenlänge in Abhängigkeit von der Konzentration der chiralen Komponente festgelegt.

Ein Maß für die Fähigkeit niedermolekularer Moleküle, in niedermolekularen Verbindungen mit nematischer Struktur helicale Überstrukturen, d. h. cholesterische Strukturen, zu induzieren, ist die »helical twisting power« (J. chem. Physics 52, 631, 1970).

Überraschenderweise wird in Analogie zu den niedermolekularen chiral-nematischen Mischsystemen die reziproke Reflexions-Wellenlänge $1/\lambda_R$ der erfindungsgemäßen Polymerphase mit zunehmendem Anteil an chiraler Komponente größer. Bei verschiedenen chiralen Komponenten ist bei gleich großem Anteil eine unterschiedliche Größe von $1/\lambda_R$ der erfindungsgemäßen Polymerphase festzustellen, was offensichtlich von der jeweiligen Konstitution der chiralen Komponente abhängig ist und deren »helical twisting power« zuzuschreiben ist. Es besteht somit bei den Polymerphasen der Erfindung die Möglichkeit, $\lambda_R$ durch Art und Konzentration der chiralen Komponente in einem weiten Bereich zu variieren.

Ein induziert cholesterisches Copolymer, das im sichtbaren Wellenlängenbereich reflektiert, sollte danach eine chirale Komponente mit hoher »helical twisting power« enthalten. Das chirale Strukturelement in diesem Copolymeren ist vorzugsweise eine $[-CH=N-CH(CH_3)C_6H_5]$-Gruppe. Wird hingegen Reflexion im infraroten Bereich verlangt, so kann die »helical twisting power« der chiralen Komponente relativ gering sein. Vorzugsweise ist das chirale Strukturelement in diesem Fall eine Alkoxygruppe mit mindestens einem asymmetrischen C-Atom, z. B. eine 2-Methylbutyloxy-Gruppe.

Eine geringe »helical twisting power« zeigen chirale Monomere der folgenden Struktur:

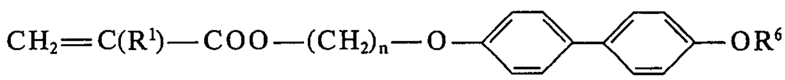

Ein induziert cholesterisches Copolymer, das diese Komponente enthält, reflektiert im infraroten Wellenlängenbereich.

Es hat sich überraschenderweise gezeigt, daß eine homogene, circular-polarisiertes Licht reflektierende Textur (Grandjean-Textur) bei der vorliegenden Polymerphase spontan ohne Anwendung äußerer Einflüsse, wie z. B. elektrische oder magnetische Felder, oberhalb der Glastemperatur $T_G$ entsteht.

Das langwelligste Absorptionsmaximum der erfindungsgemäßen induziert cholesterischen Polymerphasen kann Werte von kleiner als 350 nm zeigen, so daß eine Absorption im Sichtbaren nicht stattfindet. Unter der Bedingung, daß die Wellenlänge des reflektierten Lichtes $\lambda_R$ außerhalb des sichtbaren Wellenlängenbereiches liegt, ist die erfindungsgemäße Polymerphase vollkommen transparent, farblos und klarsichtig, und zwar in isotroper Phase (d. h. oberhalb des Klärpunktes $T_{KI}$), in flüssig-kristalliner Phase und im Glaszustand, vorausgesetzt, daß bei Temperaturen unterhalb des Klärpunktes eine einheitliche Orientierung der Phase erfolgt ist.

Der besondere Vorteil der induziert cholesterischen Polymerphase gemäß der Erfindung besteht in der weiteren Möglichkeit, die cholesterische, selektiv circular-polarisiertes Licht reflektierende Textur (Grandjean-Textur) durch Absenken der Temperatur unterhalb der Glastemperatur $T_G$, d. h. im festen Zustand, zu fixieren. Wegen dieser Eigenschaften ist die erfindungsgemäße Polymerphase neben der Verwendbarkeit auf praktisch allen technischen Gebieten, auf denen niedermolekulare cholesterische Systeme verwendet werden, geeignet, Orientierungen und damit die diesen entsprechenden Informationen nur durch Temperatursenkung unter die Glastemperatur in den Glaszustand zu übernehmen und zu speichern. Zur Anwendung als Speicherelement wird z. B. die erfindungsgemäße Polymerphase oberhalb der Glastemperatur als viskose Flüssigkeit verarbeitet und die Lage der optischen Achse durch elektrische oder magnetische Felder festgelegt. Anschließend werden Form und Struktur der Orientierung, die als Information ausgebildet sein kann, durch Abkühlen auf Temperaturen unterhalb der Glastemperatur $T_G$ fixiert.

Schließlich ergeben sich zahlreiche weitere Anwendungsmöglichkeiten wegen des Umstandes, daß die Polymerphase gemäß der Erfindung cholesterische Eigenschaften mit typischen Polymereigenschaften, wie Fähigkeit zur Schicht-, Folien- und Faserbildung, leichte Verformbarkeit usw., vereinigt. Diese Eigenschaften können in an sich bekannter Weise durch Copolymerisation oder Vermischen mit weiteren Komponenten, durch Variation der Molekulargewichte, durch Zusätze der verschiedensten anorganischen oder organischen Additive und Metalle und durch viele weitere, dem Polymerfachmann geläufige Behandlungen modifiziert werden.

Beispielsweise ist die erfindungsgemäße Polymerphase zur Herstellung von Circularpolarisations-Filtern oder selektiven Reflektoren geeignet. Bei Verwendung von zwei Filtern oder Reflektoren hintereinander, welche die gleiche Wellenlänge $\lambda_R$ reflektieren, jedoch entgegengesetzte Helixstruktur der cholesterischen Phase aufweisen, werden selektive Reflektoren erhalten. Die entgegengesetzte Helixstruktur entsteht beispielsweise durch Verwendung der optischen Antipoden der chiralen Komponente. Die Reflexionswellenlänge der selektiven Reflektoren läßt sich auch für infrarote Strahlung einstellen, wobei das Material für den übrigen Wellenlängenbereich außer den Wellenlängenbereichen der Eigenabsorption durchlässig ist. Wird dagegen die Polymerphase auf einen lichtdurchlässigen Träger mit $\lambda_R/4$-Eigenschaften gebracht, so sind selektive Linearpolarisations-Filter und Reflektoren herstellbar. Filter dieser Art finden in der Display-Technologie Anwendung (Scheffer-Zelle).

Die Monomeren

$$CH_2=C(R^1)-COO-(CH_2)_n-R^3$$

bzw.

$$CH_2=C(R^1)-COO-(CH_2)_n-R^4$$

lassen sich aus bekannten Verbindungen nach an sich bekannten Verfahren herstellen. Im folgenden werden beispielhafte Herstellungsmöglichkeiten aufgezeigt. Die Reste $R^1$, $R^2$, $R^3$, $R^4$, $R^5$ sowie m und n haben die oben aufgezeigte Bedeutung, d. h. die Monomeren, die den Rest $R^3$ aufweisen, sind nematische und Monomere mit dem Rest $R^4$ sind chirale Verbindungen.

Zur Herstellung der Monomeren der allgemeinen Formel

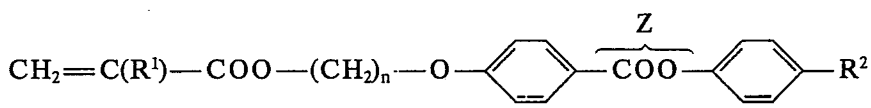

bzw.

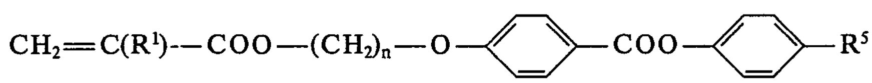

geht man aus von an sich bekannten Verbindungen der Formel

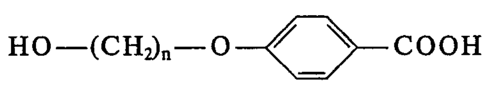

Man führt die polymerisionsfähige Acryl- oder Methacrylgruppe vorzugsweise durch azeotrope Veresterung nach bekannten Verfahren ein und erhält folgende Monomere:

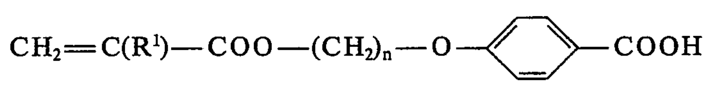

z. B.

4-(ω-Propenoyloxy-äthoxy)benzoesäure,
4-(ω-Propenoyloxy-propyloxy)benzoesäure,
4-(ω-Propenoyloxy-butyloxy)benzoesäure,
4-(ω-Propenoyloxy-pentyloxy)benzoesäure,
4-(ω-Propenoyloxy-hexyloxy)benzoesäure,
4-[ω-(2-Methylpropenoyloxy)-äthoxy]benzoesäure,
4-[ω-(2-Methylpropenoyloxy)-propyloxy]benzoesäure,
4-[ω-(2-Methylpropenoyloxy)-butyloxy]benzoesäure,
4-[ω-(2-Methylpropenoyloxy)-pentyloxy]benzoesäure,
4-[ω-(2-Methylpropenoyloxy)-hexyloxy]benzoesäure.

Die p-substituierten, polymerisationsfähigen Benzoesäuren werden nach bekannten Veresterungsverfahren mit an sich bekannten Phenolderivaten der Formel

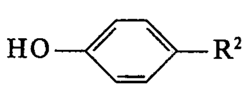

bzw.

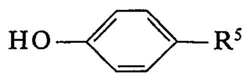

verestert. Die in der letzten Stufe eingesetzten Phenolderivate sind an sich bekannte Hydrochinonmonoalkyläther, p-Alkylphenole, p-Hydroxybenzoesäurealkylester, 4-Hydroxy-4'-alkyloxydiphenylene, 4-Hydroxy-4'-alkyldiphenylene, p-Hydroxyzimtsäurealkylester oder Azomethine. Man erhält die oben genannten Monomeren (Z = COO).

Zur Herstellung der Monomeren der allgemeinen Formel

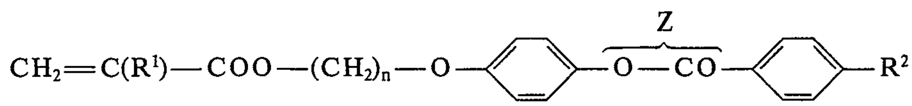

bzw.

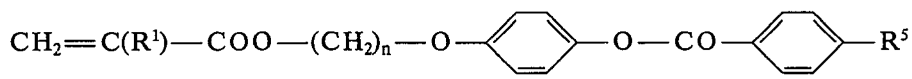

geht man aus von an sich bekannten Verbindungen der Formel

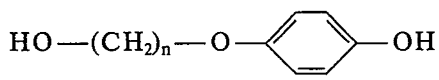

Man führt die polymerisationsfähige Acryl- oder Methacrylgruppe vorzugsweise durch azeotrope Veresterung nach bekannten Verfahren ein und erhält Monomere der allgemeinen Formel

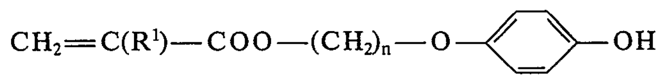

z. B.

4-(ω-Propenoyloxy-äthoxy)-phenol,
4-(ω-Propenoyloxy-propyloxy)-phenol,
4-(ω-Propenoyloxy-butyloxy)-phenol,
4-(ω-Propenoyloxy-pentyloxy)-phenol,
4-(ω-Propenoyloxy-hexyloxy)-phenol,
4-[ω-(2-Methylpropenoyloxy)-äthoxy]-phenol,
4-[ω-(2-Methylpropenoyloxy)-propyloxy]-phenol,
4-[ω-(2-Methylpropenoyloxy)-butyloxy]-phenol,
4-[ω-(2-Methylpropenoyloxy)-pentyloxy]-phenol,
4-[ω-(2-Methylpropenoyloxy)-hexyloxy]-phenol.

Diese polymerisationsfähigen Verbindungen werden nach bekannten Veresterungsverfahren mit an sich bekannten, in p-Stellung substituierten Benzoesäuren der allgemeinen Formel

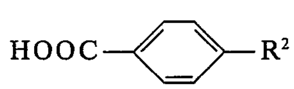

bzw.

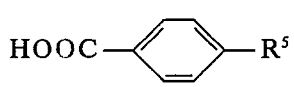

verestert. Vorzugsweise sind diese Verbindungen p-Alkyloxy-benzoesäuren oder p-Alkylbenzoesäuren. Man erhält die oben genannten Monomeren (Z = OCO).

Zur Herstellung der Monomeren der allgemeinen Formel

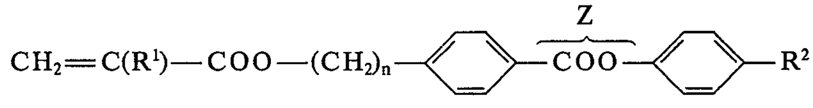

bzw.

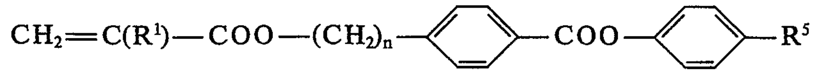

geht man aus von an sich bekannten Verbindungen der Formel

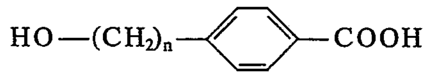

Man führt die polymerisationsfähige Acryl- oder Methacrylgruppe vorzugsweise durch azeotrope Veresterung nach bekannten Verfahren ein und erhält folgende Monomere:

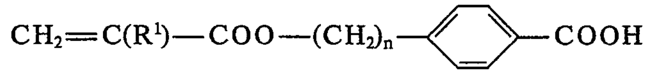

Die p-substituierten, polymerisationsfähigen Benzoesäuren werden nach bekannten Veresterungsverfahren mit an sich bekannten Phenolderivaten der Formel

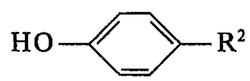

bzw.

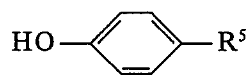

verestert. Man erhält die obengenannten Monomeren (Z = COO).

Zur Herstellung der Monomeren der allgemeinen Formel

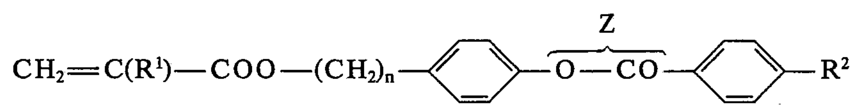

bzw.

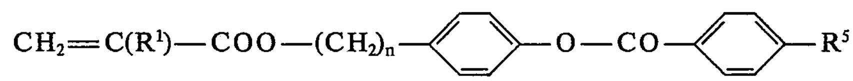

geht man aus von an sich bekannten Verbindungen der Formel

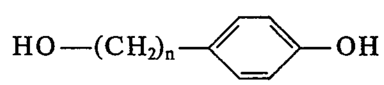

Man führt die polymerisationsfähige Acryl- oder Methacrylgruppe vorzugsweise durch azeotrope Veresterung nach bekannten Verfahren ein und erhält folgende Monomere:

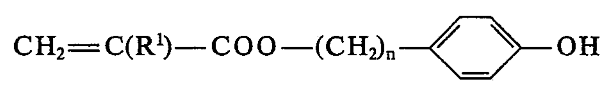

Die polymerisationsfähigen Verbindungen werden nach bekannten Veresterungsverfahren mit an sich bekannten, in p-Stellung substituierten Benzoesäuren der allgemeinen Struktur

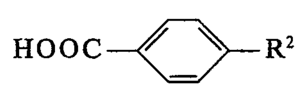

bzw.

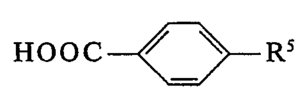

verestert. Man erhält die oben beschriebenen Monomeren (Z = OCO).

Zur Herstellung der Monomeren der allgemeinen Formel

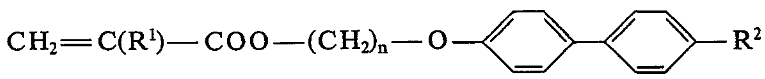

bzw.

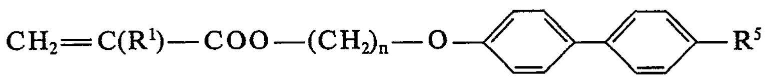

geht man aus von an sich bekannten Verbindungen der Formel

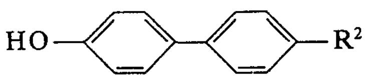

bzw.

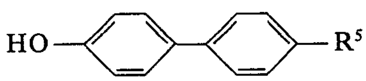

und setzt diese um mit an sich bekannten $\omega$-Hydroxy-alkylhalogeniden, z. B.

$$HO-(CH_2)_n-Cl$$

Für n = 4 oder 5 wird anstelle der $\omega$-Hydroxy-alkylhalogenide vorzugsweise mit einer $\omega$-Halogencarbonsäure, z. B

$$Cl-(CH_2)_n-COOH$$

umgesetzt, anschließend wird die Carboxylgruppe zur Hydroxygruppe reduziert.

In die erhaltene Verbindung mit der Formel

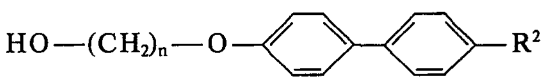

bzw.

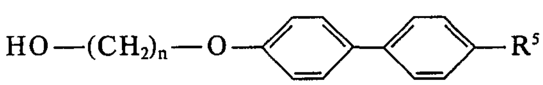

wird die polymerisationsfähige Acryl- oder Methacrylgruppe vorzugsweise durch azeotrope Veresterung nach bekannten Verfahren eingeführt. Man erhält die obengenannten Monomeren, insbesondere die chiralen Monomeren:

| | | | | |
|---|---|---|---|---|
| 4-($\omega$-Propenoyloxy | äthyloxy | | (2-methylbutyloxy)- | |
| | propyloxy | 4'- | (1-methylheptyloxy)- | biphenyl |
| 4-[$\omega$-(2-Methylpropenoyloxy) | hexyloxy | | (1-methylpropyloxy)- | |

Die Herstellung der Homo- oder Copolymeren aus den Monomeren

$$CH_2=C(R^1)-COO-(CH_2)_n-R^3$$

und

$$CH_2=C(R^1)-COO-(CH_2)_n-R^4$$

erfolgt vorzugsweise durch radikalische Polymerisation. Die Reaktion wird beispielsweise durch UV-Bestrahlung oder Radikalbildner gestartet. Die Monomeren können in Lösung oder in Substanz polymerisiert werden, ohne daß eine Phasentrennung Polymer-Monomer erkennbar ist.

Polymer und Monomer sind beliebig mischbar, und damit die Eigenschaften des Polymerisats variierbar. Das chirale Monomere kann selbst mesomorph, d. h. flüssig-kristallin sein, diese Eigenschaft ist jedoch nicht unbedingt erforderlich. Bei chiralen, selbst nicht mesomorphen Comonomeren darf jedoch eine bestimmte Grenzkonzentration nicht überschritten werden, da dann die mesomorphe Phase zerstört wird. Die Grenzkonzentration, bis zu deren Erreichen die cholesterische Phase erhalten bleibt, hängt von der Struktur der chiralen Komponente ab.

Zur Herstellung von Gemischen wird das aus dem Monomeren hergestellte nematogene Homopolymer und das chirale Homopolymer gemeinsam z. B. in einem organischen Lösungsmittel gelöst und einer Gefriertrocknung unterzogen. Das vom Lösungsmittel befreite Gemisch der beiden Homopolymere zeigt in der flüssig-kristallinen Phase eine induziert cholesterische Struktur. In gleicher Weise wird ein Gemisch aus einem nematischen Homopolymer mit einer niedermolekularen chiralen Verbindung hergestellt, wobei die Struktur der niedermolekularen Komponente so gewählt werden muß, daß beide Komponenten mischbar sind.

Die Erfindung wird anhand der folgenden Beispiele näher erläutert. In den Beispielen 1 und 2 werden die bevorzugten nematogenen Monomeren beschrieben, in Beispiel 3 und 4 die bevorzugten chiralen Monomeren, in Beispiel 5 ein induziert cholesterisches Copolymer und in Beispiel 6 eine induziert cholesterische Mischung.

## Beispiel 1

Im folgenden wird die Herstellung von verschiedenen Monomeren gemäß der allgemeinen Formel

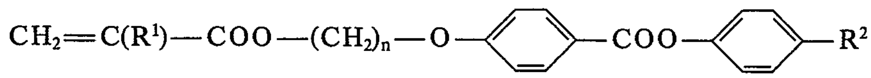

beschrieben, in der

$R^1$ Wasserstoff oder eine Methylgruppe
n 2, 3 oder 6 und
$R^2$ $-O-(CH_2)_mH$ m = 1 oder 2

$-CH_3$ 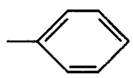

oder

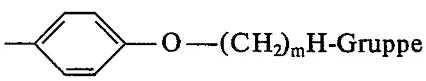 m = 1 oder 2

ist.

1.1. Ausgehend von an sich bekannten Verbindungen der Struktur

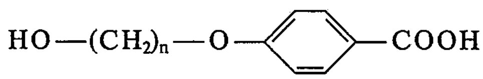 n = 2, 3 oder 6

wird die polymerisationsfähige Acryl- oder Methacrylgruppe azeotrope Veresterung nach bekannten Verfahren eingeführt:

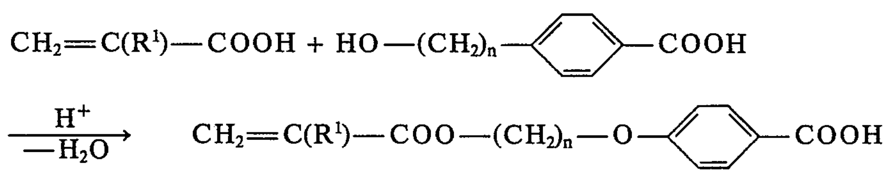


1.1.1. Darstellung von 4-(2-Propenoyloxy)äthoxy-benzoesäure

50 g p-(2-Hydroxyäthoxy)-benzoesäure, 120 g Acrylsäure, 5 g p-Toluolsulfonsäure und 5 g Hydrochinon als Inhibitor werden in 200 ml Chloroform in einem 500-ml-Kolben mit Wasserabscheider und Rückflußkühler 5 h unter Rückfluß gekocht. Danach wird die abgekühlte Reaktionslösung in ca. 1 l Äther gegeben und 5 × mit ca. 200 ml Wasser ausgewaschen. Nach Trocknen der Ätherphase mit $Na_2SO_4$ werden die Lösungsmittel abgezogen und das Produkt in wenig Äthanol gelöst und so viel Petroläther zugegeben, bis die Lösung gerade klar bleibt. Beim Abkühlen auf −15° C fällt das Produkt aus. Die Umkristallisation wird bis zur dünnschichtchromatographischen Reinheit der Substanz wiederholt (Kieselgel, Laufmittel Essigester : Hexan/ 4 : 1).

Analog wurde die

4-(3-Propenoyloxy)propyloxy-benzoesäure und die 4-(6-Propenoyloxy)hexyloxy-benzoesäure

hergestellt.

1.1.2. Darstellung von 4-[2-(2-Methylpropenoyloxy)äthoxy]-benzoesäure

50 g p-(2-Hydroxyäthoxy)-benzoesäure, 130 g Methacrylsäure, 5 g p-Toluolsulfonsäure und 5 g Hydrochinon als Inhibitor werden in 200 ml $CHCl_3$ gelöst und in einem 500-ml-Kolben mit Wasserabscheider und Rückflußkühler so lange unter Rückfluß gekocht, bis etwa 6 ml Wasser abgeschieden sind (ca. 20 h). Die abgekühlte Lösung wird in ca. 1 l Äther gegeben und 5 × mit ca. 200 ml Wasser ausgewaschen. Anschließend werden nach Trocknung mit $Na_2SO_4$ die Lösungsmittel abgezogen und das Rohprodukt wie unter 1.1.1. beschrieben umkristallisiert.
Analog wurden hergestellt

4-[3-(2-Methylpropenoyloxy)propyloxy]-benzoesäure und die 4-[6-(2-Methylpropenoyloxy)hexyloxyl]-benzoesäure.

1.2.1. Die unter 1.1.1. und 1.1.2. dargestellten p-substituierten, polymerisationsfähigen Benzoesäuren werden nach bekannten Veresterungsverfahren mit an sich bekannten Hydrochinonmonoalkyläthern der allgemeinen Struktur

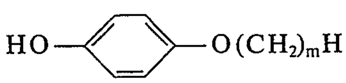

$m = 1$ oder 2

zu Verbindungen der Struktur

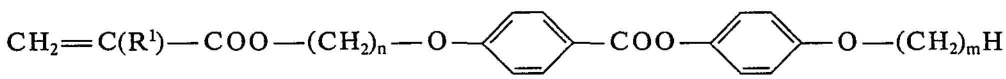

$n = 2, 3, 6$

verestert, wobei Verbindungen mit Summe $m + n$ größer als 8 zu smektischen Polymeren führen.

Variante A am Beispiel von
4-[2-(2-Methylpropenoyloxy)äthoxy]-benzoesäure-(4′-methoxy)phenylester ($n = 2$; $m = 1$)

4,6 g Natrium werden in 50 ml absolutem Äthanol in einem 100-ml-Kolben gelöst und anschließend 24,0 g Hydrochinonmonomethyläther zugegeben. Die Lösung wird auf −15°C abgekühlt und langsam das aus 22,7 g 4-[2-(2-Methylpropenoyloxy)äthoxy]-benzoesäure und 40 ml Thionylchlorid hergestellte Säurechlorid unter Rühren zugetropft. Die Lösung wird etwa 4 h bei −15°C, dann über Nacht bei Zimmertemperatur gerührt. Der Ansatz wird anschließend in 500 ml Äther aufgenommen, 1 × mit wäßriger $NaHCO_3$-Lösung und 3 × mit 100 ml Wasser ausgewaschen. Die Lösungsmittel werden nach dem Trocknen mit $Na_2SO_4$ abgezogen.

Variante B am Beispiel von
4-(2-Propenoyloxy)äthyloxy-benzoesäure-(4′-methoxy)phenylester

11,8 g 4-(2-Propenoyloxy)äthyloxy-benzoesäure, 6,2 g Hydrochinonmonomethyläther und wenige mg 1,3-Dinitrobenzol als Inhibitor werden in 50 ml absolutem THF und 25 ml $CH_2Cl_2$ in einem 250-ml-Kolben mit Tropftrichter und Trockenrohr ($CaCl_2$) gelöst und mit Eis abgekühlt. Zu dieser Lösung wird unter Rühren langsam 11,3 g Dicyclohexylcarbodiimid, gelöst in 20 ml $CH_2CL_2$, zugetropft. Das Gemisch wird unter Rühren ca. 3 h bei 0°C, dann bei Zimmertemperatur über Nacht stehengelassen. Anschließend wird ausgefallener Dicyclohexylharnstoff abfiltriert und die Lösung eingeengt. Mit Acetonitril aufgenommen, wird erneut ausgefallener Harnstoff abfiltriert. Das Acetonitril wird anschließend abgezogen.

Aufbereitung für Variante A und Variante B

Die Rohprodukte werden mit Essigsäureäthylester aufgenommen (ca. 20%ige Lösungen) und zur Kristallisation auf −15°C abgekühlt. Die ausgefallenen Produkte werden abgesaugt. Bei tiefschmelzenden Verbindungen, bevorzugt den Acrylverbindungen, ist mit einer gekühlten Nutsche abzusaugen. Die Umkristallisation wird so oft wiederholt, bis dünnschichtchromatographisch keine Verunreinigungen zu erkennen sind (Kieselgel, Laufmittel Hexan : Essigsäureäthylester/1 : 4).

Auf analoge Weise wurde unter der Bedingung $n = 6$, $m = 1$ das folgende Monomer hergestellt, welches ein nematisches Polymer bilden kann.

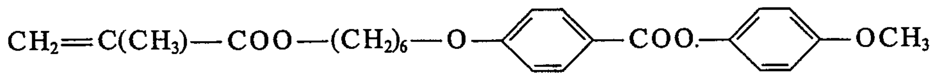

1.2.2. Die unter 1.1.2. dargestellte p-substituierte, polymerisationsfähige 4-[6-(2-Methylpropenoyloxy)-hexyloxy]-benzoesäure wurde nach bekannten Veresterungsverfahren mit an sich bekannten 4-Methylphenol zur Verbindung der Struktur

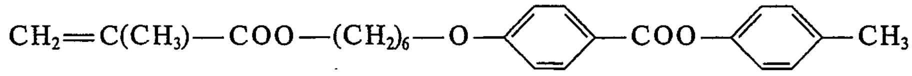

verestert.
Die Veresterung und Aufarbeitung wird nach der in Punkt 1.2.1. angegebenen Methode durchgeführt.

1.2.3. Die unter 1.1.2. dargestellte p-substituierte, polymerisationsfähige 4-[2-(2-Methylpropenoyloxy)-äthoxy]-benzoesäure wurde nach bekannten Veresterungsverfahren mit an sich bekanntem 4-Hydroxy-4'-äthoxy-biphenyl der Struktur

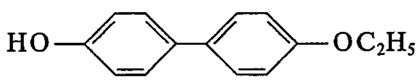

zur Verbindung der Struktur

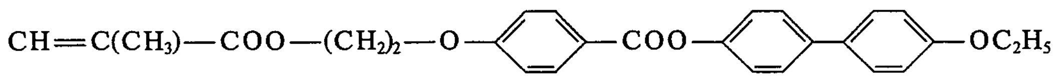

verestert.
Die Veresterung kann nach Variante A oder Variante B, beschrieben unter 1.2.1., durchgeführt werden. Das Produkt wurde nach 1.2.1. aufgearbeitet.
Analog wurde hergestellt mit 4-Hydroxy-4'-methoxy-biphenyl

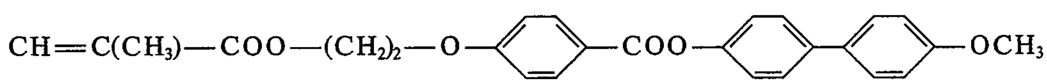

Die analoge Veresterung der unter 1.1.2. dargestellten 4-[6-(2-Methylpropenoyloxy)hexyloxy]-benzoesäure mit 4-Hydroxy-4'-methoxy-biphenyl ergab das folgende Monomere

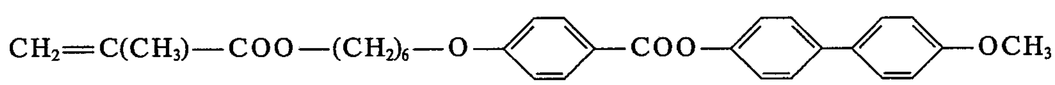

1.2.4. Die unter 1.1.1. bzw. 1.1.2. dargestellten p-substituierten, polymerisationsfähigen Benzoesäuren:
4-[3-(2-Methylpropenoyloxy)propyloxy]-benzoesäure
4-[6-(2-Methylpropenoyloxy)hexyloxy]-benzoesäure und
4-(6-Propenoyloxy-hexyloxy)benzoesäure
wurden nach bekannten Veresterungsverfahren mit an sich bekanntem p-Hydroxy-biphenyl

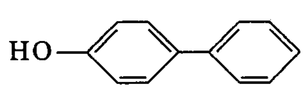

zu Verbindungen der Struktur

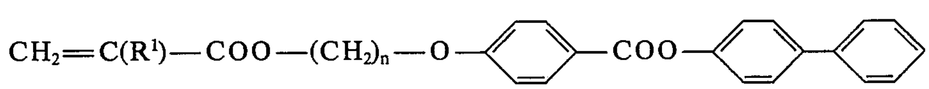

$R^1 = H$ $n = 6$

$R^1 = CH_3$ $n = 3$ oder 6

verestert.
Die Veresterung kann nach Variante A oder Variante B, beschrieben unter 1.2.1., durchgeführt werden. Die Produkte wurden nach 1.2.1. aufgearbeitet.
Bei den zuletzt genannten Monomeren, für die $R^1 = H$, $n = 6$ und $R^1 = CH_3$, $n = 6$ entspricht, ist die

Methylgruppe in der polymerisationsfähigen Methacrylgruppe durch Wasserstoff ersetzt, bei sonst gleicher Struktur der mesogenen Gruppen. Die aus den beiden Monomeren hergestellten Polymerphasen zeigen das gleiche flüssig-kristalline Verhalten bei verschobenen Phasenübergängen. Dieses Verhalten wurde allgemein bei analogen Verbindungen gemäß der Erfindung festgestellt.

## Beispiel 2

2.1. Im folgenden wird die Herstellung von einigen erfindungsgemäßen Monomeren gemäß der folgenden Formel

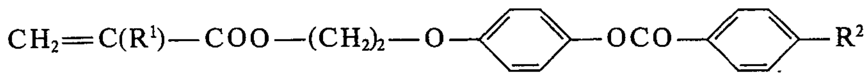

beschrieben, in der $R^1$ Wasserstoff oder eine Methylgruppe ist und $R^2$ die obengenannte Bedeutung hat. Ausgehend von an sich bekannten Verbindungen der Struktur

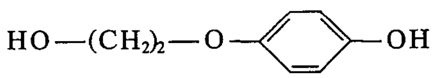

wird die polymerisationsfähige Acryl- oder Methacrylgruppe durch azeotrope Veresterung nach bekannten Verfahren eingeführt

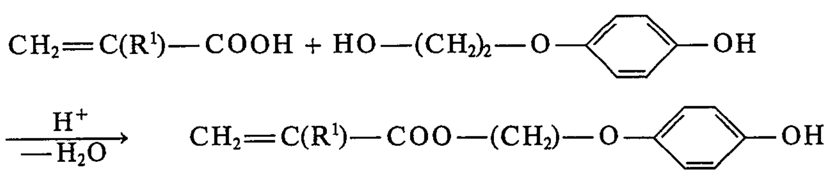


2.1.1. Darstellung von 4-(2-Propenoyloxy-äthoxy)-phenol

50 g p-(Hydroxyäthoxy)-phenol, 110 g Acrylsäure, 5 g p-Toluolsulfonsäure und 5 g Hydrochinon als Inhibitor werden in 200 ml Chloroform in einem 500-ml-Kolben mit Wasserabscheider und Rückflußkühler 5 h unter Rückfluß gekocht. Danach wird die abgekühlte Reaktionslösung in ca. 1 l Äther gegeben und 5 × mit ca. 200 ml Wasser ausgewaschen. Nach Trocknen der Ätherphase mit $Na_2SO_4$ werden die Lösungsmittel abgezogen und das Produkt in Äthanol gelöst und so viel Petroläther zugesetzt, bis die Lösung gerade klar bleibt. Beim Abkühlen auf −15°C fällt das Produkt aus. Die Umkristallisation wird bis zur dünnschichtchromatographischen Reinheit der Substanz wiederholt (Kieselgel, Laufmittel Essigester : Hexan/4 : 1).

2.1.2. Darstellung von 4-[2-(2-Methylpropenoyloxy)äthoxy]-phenol

50 g p-(Hydroxyäthoxy)-phenol, 120 g Methacrylsäure, 5 g p-Toluolsulfonsäure und 5 g Hydrochinon als Inhibitor wurden in 200 ml $CHCl_3$ gelöst und in einem 500-ml-Kolben mit Wasserabscheider und Rückflußkühler so lange unter Rückfluß gekocht, bis etwa 5 ml Wasser abgeschieden sind (ca. 20 h). Die abgekühlte Lösung wird in ca. 1 l Äther gegeben und 5 × mit ca. 200 ml Wasser ausgewaschen. Anschließend werden nach Trocknung mit $Na_2SO_4$ die Lösungsmittel abgezogen und das Rohprodukt wie unter 1.1.1. beschrieben umkristallisiert.

2.1.3. Die unter 2.1.1. und 2.1.2. dargestellten p-substituierten polymerisationsfähigen Phenole werden nach bekannten Veresterungsverfahren mit an sich bekannten p-substituierten Benzoesäuren der allgemeinen Struktur

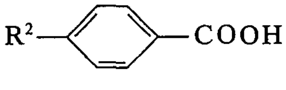

zu Verbindungen der Struktur

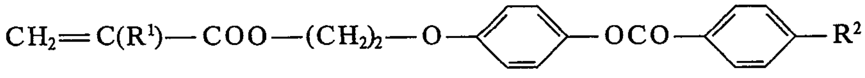

verestert.

2.2. Die Herstellung des erfindungsgemäßen Monomeren der Formel

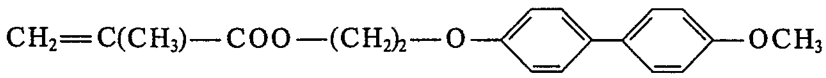

erfolgt nach in 4. beschriebenen Reaktionsbedingungen nach folgendem Schema:

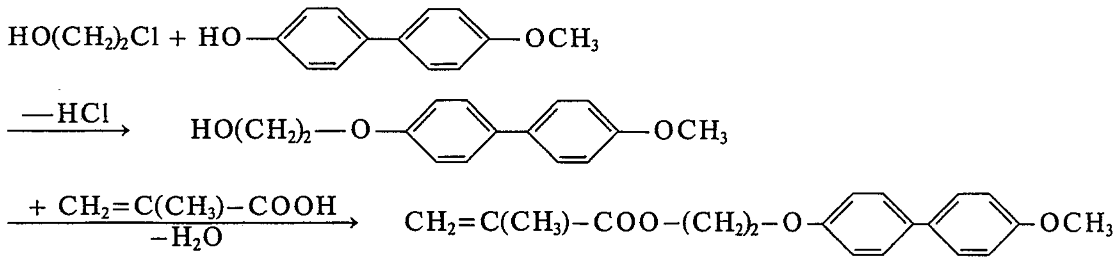


Beispiel 3

Im folgenden wird die Herstellung des chiralen Monomeren (d- oder l-Antipode) der Formel

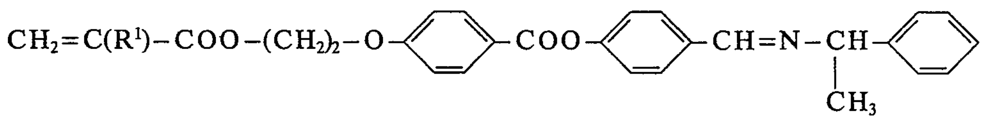

$R^1$ = H oder $CH_3$, und

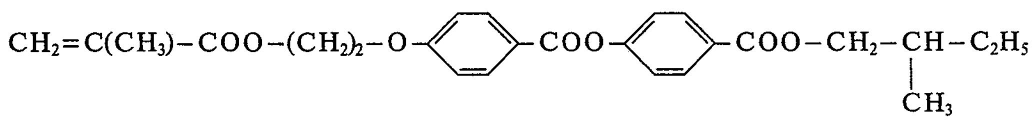

beschrieben. Diese Monomeren bestehen aus einer polymerisationsfähigen Gruppe, einem zur nematogenen Copolymerisationskomponente analog gebauten Strukturteil und dem bevorzugten chiralen Strukturelement.

Man geht aus von den unter 1.1.1. und 1.1.2. beschriebenen Benzoesäuren

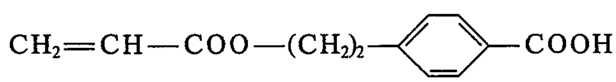

bzw.

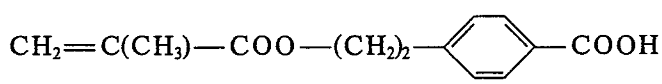

und erhält nach bekannten Verfahren durch Veresterung mit den chiralen Phenolen (d- oder l-Antipode)

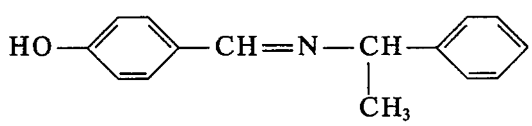

bzw.

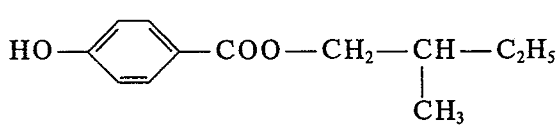

die obengenannten Monomeren, z. B.

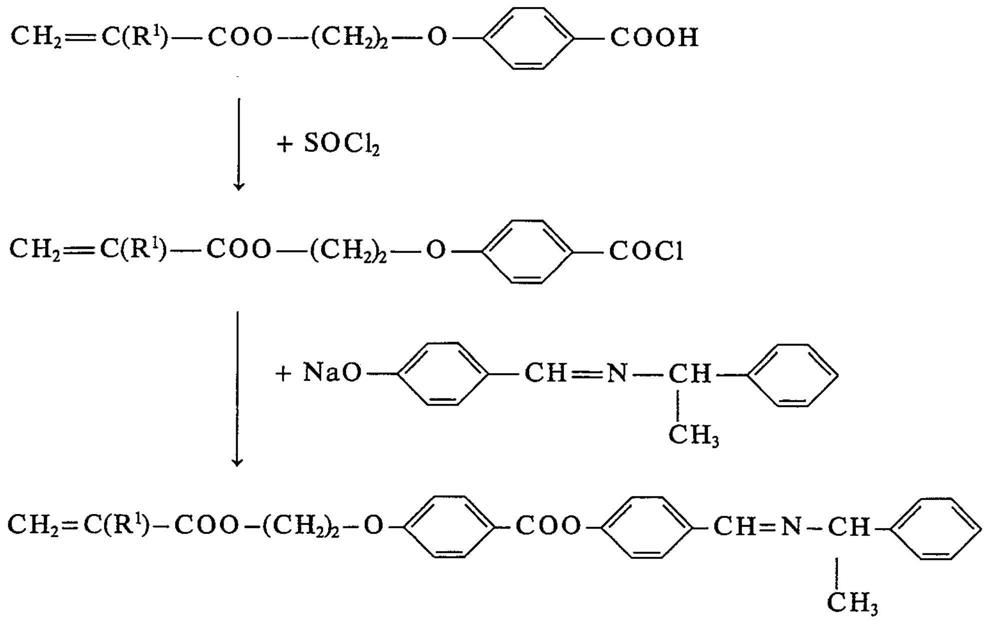


3.1.1. Darstellung von 4-Hydroxybenzyliden-1-phenyläthylimin

Zu einer heißen Lösung von 15 g p-Hydroxybenzaldehyd in 150 ml Toluol werden 16 ml d-1-Phenyläthylamin (bzw. die l-Komponente) zugetropft, wobei die Reaktion zur Schiffschen Base sofort eintritt. Entstehendes Wasser wird azeotrop abdestilliert (Wasserabscheider). Ist die äquimolare Menge Wasser abgetrennt, läßt man die Lösung abkühlen, saugt das Reaktionsprodukt ab und kristallisiert es aus Äthanol um (Fp. 170° C Zers.).

3.1.2. Darstellung von 4-[4-(2-Propenoyloxyäthoxy)benzoyloxy]-benzyliden-1-phenyläthyl-imin

a. Herstellung der Phenolatlösung:
   42 mMol Natrium werden unter Feuchtigkeitsausschluß zu 40 ml Äthanol abs. gegeben. Nach vollständigem Umsatz des Natriums werden 42 mMol der nach 3.1.1. dargestellten Schiffschen Base zugegeben.
b. 42 mMol 4-(ω-Propenoyloxy-äthoxy)-benzoesäure werden mit 25 ml Thionylchlorid und zwei Tropfen Dimethylformamid bei Zimmertemperatur versetzt und ca. 5 h stehengelassen (HCl- und $SO_2$-Entwicklung). Anschließend wird überschüssiges Thionylchlorid im Vakuum vollständig entfernt. Das zurückbleibende Säurechlorid wird in 20 ml Äther abs. aufgenommen und bei Raumtemperatur langsam zur Phenolatlösung unter Rühren zugetropft. Nach vollständiger Zugabe des Säurechlorids wird 2 h gerührt. Anschließend werden zum Reaktionsansatz 250 ml Äther gegeben und die ätherische Lösung 3 × mit Wasser ausgewaschen, mit $Na_2SO_4$ getrocknet und das Lösungsmittel abgezogen. Das Rohprodukt wird aus Äthanol umkristallisiert.
   Analog wird das 4-{4-[2-(2-Methylpropenoyloxy)äthoxy]benzoyloxy}-benzyliden-1-phenyl-äthylimin (d- oder l-Antipode) aus 4-[2-(2-Methylpropenoyloxy)äthoxy]-benzoesäure und dem oben genannten chiralen Phenol (d- oder l-Antipode) hergestellt.

3.2. Die Herstellung von

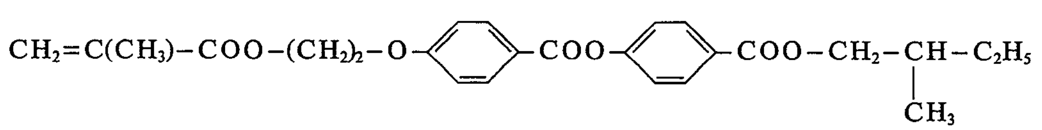

erfolgt nach der in Punkt 1.2.1. beschriebenen Veresterungs- und Aufarbeitungsmethode.

## Beispiel 4

Im folgenden wird die Herstellung des chiralen Monomeren (d- oder l-Antipode) der Formel

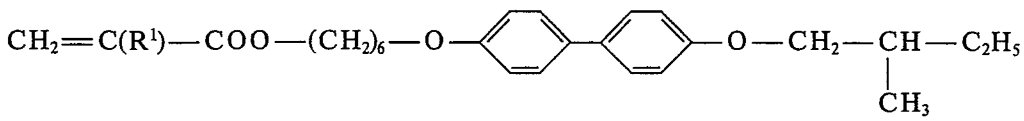

beschrieben ($R^1$ = H oder $CH_3$). Diese Monomere besteht aus einer polymerisationsfähigen Gruppe, einem zur nematogenen Copolymerisationskomponente analog gebauten Strukturteil und dem bevorzugten chiralen Strukturelement.

Die Synthese erfolgt nach folgendem Reaktionsschema:

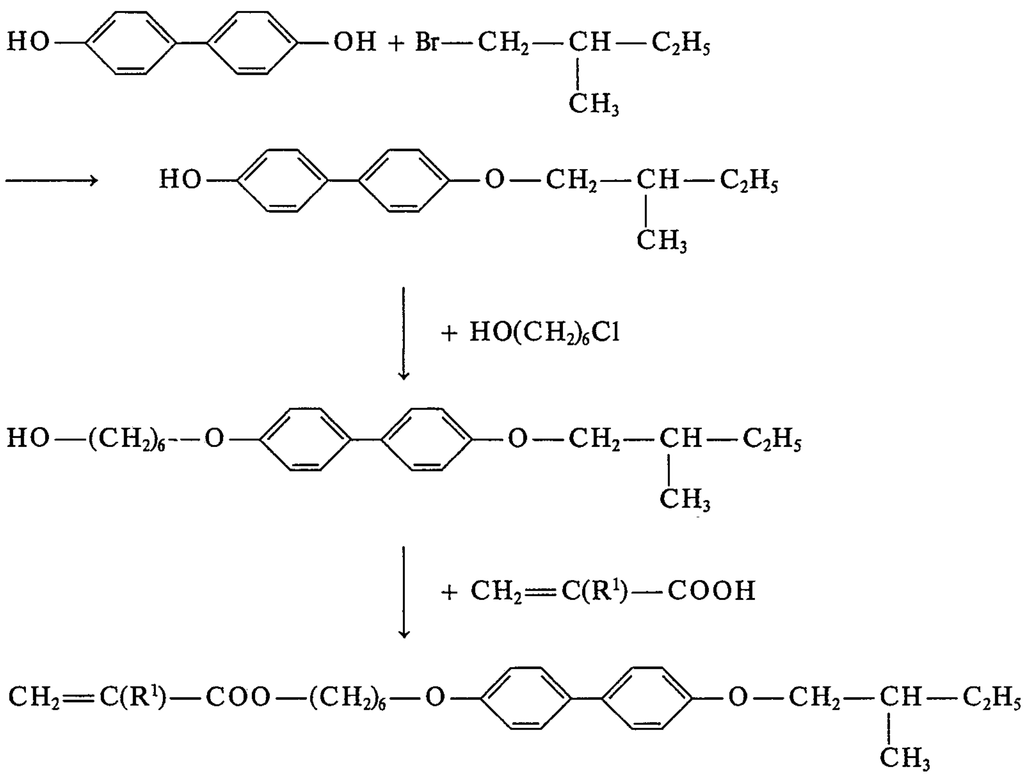


4.1. Darstellung von 4-(2-Methylbutyloxy)-4'-hydroxybiphenyl

Unter Feuchtigkeitsausschluß werden in 300 ml Äthanol abs. vorsichtig 0,5 Mol (11,5 g) Natrium gegeben. Ist das Natrium gelöst, werden 0,5 Mol (93 g) 4,4'-Dihydroxybiphenyl zugegeben. Der Reaktionsansatz wird unter Rückfluß zum Sieden erhitzt und unter kräftigem Rühren langsam 0,5 Mol (75,5 g) optisch aktives 1-Brom-2-methylbutan zugetropft. Nach vollständiger Zugabe wird ca. 5 h weiter unter Rückfluß gekocht. Nach Abkühlen der Reaktionslösung wird vom ausgefallenen NaBr abdekantiert, das Äthanol abgezogen und das Rohprodukt in 10%ige wäßrige Natronlauge gegeben und zum Sieden erhitzt. Der als Nebenprodukt entstandene Di-Äther ist in der Siedehitze unlöslich und wird durch Filtration abgetrennt. Beim Abkühlen des Filtrates fällt das Natriumsalz des Monoäthers aus. Es wird abfiltriert und noch einmal aus 10%iger NaOH umkristallisiert. Anschließend löst man das Na-Salz in Wasser und fällt das Phenol durch Ansäuern mit HCl aus. Es wird filtriert, mit Wasser ausgewaschen und getrocknet (Fp. 135° C).

4.2. Darstellung von 4-(6-Hydroxyhexyloxy)-4'-(2-Methylbutyloxy)-biphenyl

In 150 ml Wasser/Äthanol (1 : 1) werden 4,5 g KOH und 15 g 4-Hydroxy-4'-(2-Methylbutyloxy)-biphenyl gelöst, zum Sieden erhitzt und unter Rühren 9 g 6-Chlor-1-hexanol langsam zugetropft, dann ca. 10 h unter Rückfluß gekocht. Die abgekühlte Reaktionslösung wird mit HCl angesäuert und das Produkt im Scheidetrichter mit $CHCl_3$ extrahiert. Anschließend zieht man das $CHCl_3$ ab und kristallisiert das Rohprodukt aus Äthanol um (Fp. 118° C).

4.3. 4-[6-(2-Methylpropenoyloxy)-hexyloxy]-4′-(2-methylbutyloxy)-biphenyl

In 100 ml $CHCl_3$– werden 6,8 g des unter 4.2. hergestellten Biphenylderivats, 10,5 g Methacrylsäure, 0,5 g p-Toluolsulfonsäure und 0,5 g Hydrochinon als Inhibitor gelöst und ca. 20 h mit einem Wasserabscheider unter Rückfluß gekocht. Anschließend wird die Reaktionslösung in 100 ml Äther aufgenommen, mit gesättigter $Na_2CO_3$-Lösung mehrmals ausgewaschen (bis keine Färbung der wäßrigen Phase mehr auftritt), mit $Na_2SO_4$ getrocknet und das Lösungsmittel abgezogen. Das Rohprodukt wird aus Äthanol umkristallisiert. Phasenübergänge k 42,5 s 49 i.
Analog wird 4-[6-(Propenoyloxy)-hexyloxy]-4′-(2-methylbutyloxy)-biphenyl hergestellt.

Die in den Beispielen 1 bis 4 dargestellten Monomeren wurden durch Substanzanalyse sowie IR- und NMR-spektroskopisch identifiziert.

## Beispiel 5

Es wird die Herstellung eines cholesterischen Copolymeren aus dem chiralen Monomer der Struktur

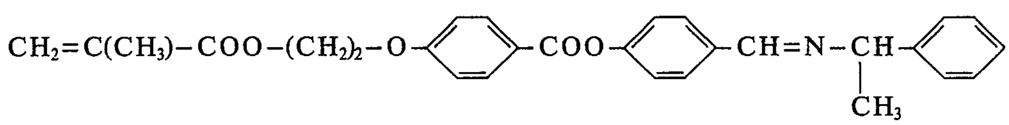

und dem nematogenen Monomeren der Struktur

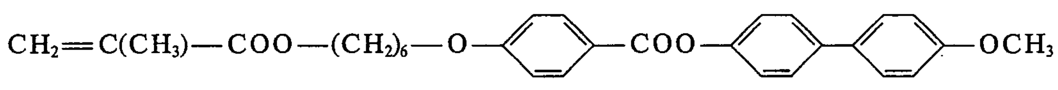

beschrieben.

5.1. Die beiden Monomeren werden radikalisch in Substanz polymerisiert. Eine Mischung der beiden Monomeren mit einem Anteil von maximal 30 Mol.% des chiralen Monomeren wird in einer Kugelmühle mit 1 Mol.% Azoisobuttersäuredinitril (AIBN) gemahlen und zwischen zwei Glasplättchen unter Sauerstoffausschluß oberhalb des Schmelzpunktes des Gemisches, jedoch immer oberhalb 55° C, erhitzt. Nach beendeter Polymerisation, frühestens jedoch nach 10 min wird die Probe abgekühlt, wobei sie beide Glasplättchen fest verbindet. Wird in diesem Zustand die Temperatur über die Glastemperatur des Copolymeren erhöht, gelangt man in den Mesophasenbereich des Copolymeren. Die Substanz ist flüssig-kristallin und die Textur bzw. die Orientierung der Moleküle, vorzugsweise wenige Grade unterhalb des Klärpunktes, durch äußere Einflüsse (Temperatur, elektrische und magnetische Felder, Druck) veränderbar. Durch Abkühlen ist der hergestellte Orientierungszustand im Glaszustand fixierbar.

5.2. Das chirale Monomer und das nematogene Monomer werden radikalisch in Lösung copolymerisiert. Hierbei werden vorzugsweise 10%ige Lösungen der Monomeren in absolutem Benzol oder Tetrahydrofuran und max. 5% Initiator unter Sauerstoffausschluß bei 55° bis 60° C ca. 20 h temperiert. Anschließend wird das Copolymere durch Zugabe der 10fachen Menge Methanol oder Aceton, bezogen auf die vorhergehende Lösung, ausgefällt, abzentrifugiert und getrocknet. Zur Reinigung wird das Copolymer 2 × umgefällt.
Das aus der Lösung umgefällte Copolymer ist amorph und zeigt keine flüssig-kristalline Struktur. Wird es beispielsweise zwischen Glasplättchen oder als Film auf einen Träger gebracht und oberhalb der Glastemperatur, jedoch unterhalb der Klärtemperatur, erwärmt, bildet sich spontan die cholesterische Grandjean-Textur aus. Sie kann durch Abkühlen unterhalb der Glastemperatur im Glaszustand des Copolymeren fixiert werden.

Im folgenden werden die optischen Eigenschaften von vier nach 5.1. oder 5.2. erhaltenen Copolymeren beschrieben, die sich durch den Anteil der chiralen Komponente unterscheiden. Die Wellenlänge $\lambda_R$ der Reflexion von circular-polarisiertem Licht ist spektroskopisch bei einer auf den Klärpunkt $T_{Kl}$ reduzierten Temperatur $T^+ = T_{Mess}/T_{Kl} = 0,9$ in der flüssig-kristallinen Phase bestimmt worden. Die Art des circular-polarisierten Lichtes ist durch Circularpolarisationsfilter ermittelt worden.
Die cholesterischen Copolymere reflektieren linkscircular-polarisiertes Licht bei Verwendung des chiralen Bausteines (–)-(1)-Phenyläthylamin im chiralen Comonomer.

| $X_{chiral}$ | $T_{KI}$ (°C) | $\lambda_R$ nm ($T^+ = 0{,}9$) |
|---|---|---|
| 11 | 245 | 1260 |
| 17 | 229 | 710 |
| 21 | 216 | 560 |
| 25 | 203 | 465 |

$X_{chiral}$ = Molenbruch des chiralen Comonomeren im Monomergemisch vor der Polymerisation.

Die Messung von $\lambda_R$ im durchfallenden Licht (Verwendung eines transparenten Trägers für den Polymerfilm) ist die Halbwertszeit von $\lambda_R$ in dem oben aufgeführten Beispiel im Bereich von 80 bis 350 nm, je nach Präparation und Schichtdicke der vermessenen Probe. Durch rasches Abkühlen der Probe unterhalb der Glastemperatur des Copolymeren kann die reflektierende Textur im Glaszustand fixiert werden.

Beispiel 6

Man stellt eine induziert cholesterische Mischung aus einem nematischen Homopolymer der Struktur

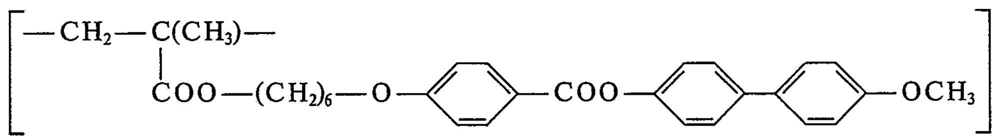

und einem chiralen Homopolymer der Struktur

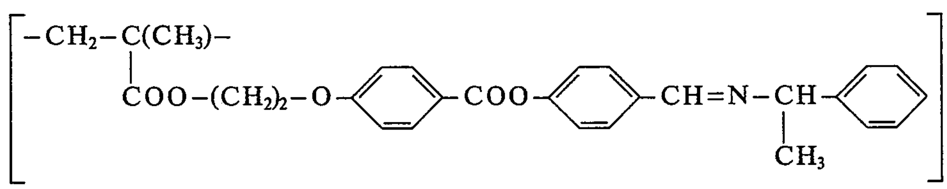

her.

Die Herstellung der beiden Homopolymeren aus den Monomeren erfolgt durch radikalische Polymerisation in Substanz oder in Lösung analog der in 5.1. und 5.2. beschriebenen Verfahren.

6.1. Das nematogene Monomere wird wie folgt radikalisch in Substanz polymerisiert:

Das mit 1 Molprozent AIBN in der Kugelmühle vermischte Monomer wird unter Sauerstoffausschluß zwischen zwei Glasplatten präpariert und auf Temperaturen oberhalb seines Schmelzpunktes (jedoch immer oberhalb 60°C) erwärmt. Bei polarisations-mikroskopischer Betrachtung ist nach etwa 3 min erkennbar, daß das Reaktionsgemisch optisch anisotrope, flüssig-kristalline Eigenschaften aufweist. Bei weiterem Fortschreiten der Polymerisation ist, je nach Monomertyp nach etwa 10 min, die klare anisotrope Substanz ausgehärtet und verbindet fest beide Glasplatten. Wird in diesem Zustand die Temperatur über die Glastemperatur des Homopolymeren erhöht, gelangt man in dessen mesophasen Bereich. Die Substanz ist flüssig-kristallin und die Textur bzw. die Orientierung der Moleküle durch äußere Einflüsse (elektrische und magnetische Felder, Druck) veränderbar. Durch Abkühlung ist der hergestellte Orientierungszustand im Glaszustand fixierbar.

6.2. Das nematogene Monomere wird radikalisch in Lösung polymerisiert. Hierbei werden 10%ige Lösungen des Monomeren in absolutem Benzol mit 1%, 2% bzw. 4% AIBN versetzt und unter Sauerstoffausschluß 20 h bei 60°C temperiert. Anschließend wird das Homopolymer aus der 10fachen Menge Methanol (bezogen auf die benzolische Lösung) ausgefällt, abzentrifugiert und getröcknet. Zur Reinigung wird Homopolymerisat 2 × umgefällt.

Wird analog das Monomere

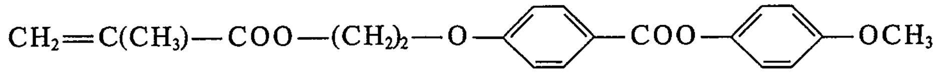

auf diese Weise polymerisiert, wurde hinsichtlich des Einflusses der Initiatorkonzentration auf den Mesophasenbereich der Polymeren festgestellt, daß letzterer von der Initiatorkonzentration weitgehend unabhängig ist, wie auch aus der folgenden kleinen tabellarischen Übersicht hervorgeht:

| Initiator | $T_{KI}$ | $T_g$ |
|---|---|---|
| 1% | 123 | 101 |
| 2% | 122,5 | 98 |
| 4% | 120 | 96 |

In der folgenden Tabelle sind die Phasenübergänge einiger Homopolymerer zusammengestellt, die aus einem Monomeren der Struktur

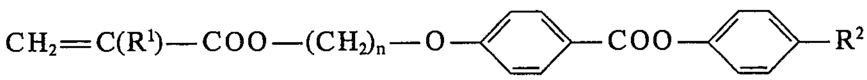

bestehen:

| $R^1$ | n | $R^2$ | Phasenübergänge (°C) |
|---|---|---|---|
| $CH_3$ | 2 | $-OCH_3$ | g 100 n 121 i |
| $CH_3$ | 6 | $-OCH_3$ | g 95 n 105 i |
| $CH_3$ | 6 | $-CH_3$ | g 75 n 84 i |
| $CH_3$ | 2 | 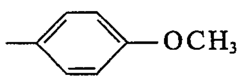 | g 120 n 177 i |
| $CH_3$ | 6 | 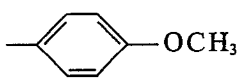 | g 60 s 133 n 271 i |
| $CH_3$ | 3 | 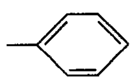 | g ca. 90 s 176 n 187 i |
| $CH_3$ | 6 | 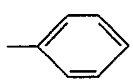 | g ca. 70 s 164 n 184 i |
| H | 6 | 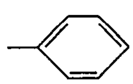 | g ca. 70 s 176 n 191 i |
| $CH_3$ | 2 | 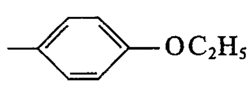 | g s 212 n 300 i |

Phasenübergänge des Homopolymers aus Monomeren der Struktur

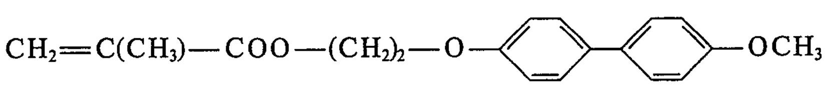

g 120 n 150 i

Die Enthalpien für den jeweiligen Phasenübergang flüssig-kristallin nach isotrop wurden durch DSC-Messung ermittelt.
Die folgenden auf analoge Weise aus ihren Monomeren hergestellten chiralen Homopolymeren zeigen folgende Eigenschaften

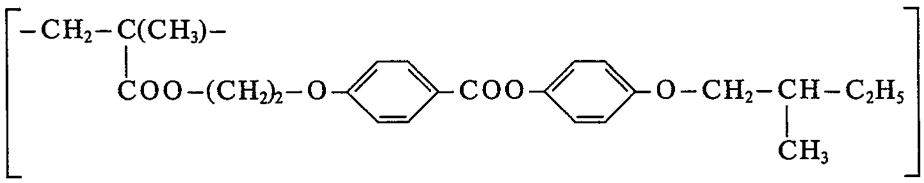

$T_{KI}$ 140°C $T_g$ ca. 95°C $\Delta H$(cal/g) 2,6 (smektisch)

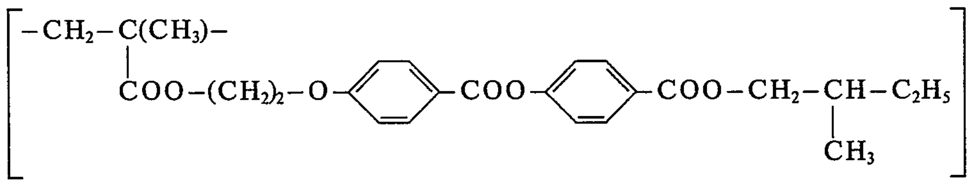

$T_{KI}$ – $T_g$ ca. 95°C $\Delta H$(cal/g) – (isotrop)

6.3. Es werden 20 Mol-% (bezogen auf die Monomereinheit) Homopolymer der Verbindung

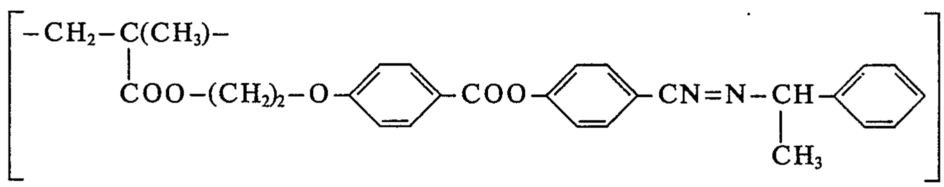

mit 80 Mol-% des nematischen Homopolymeren

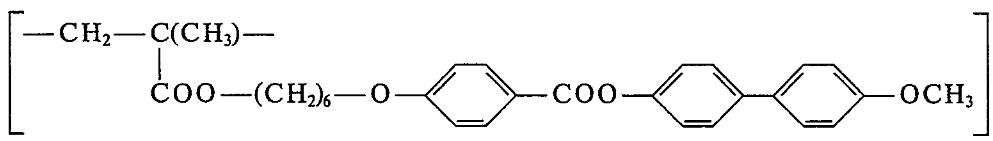

in Dioxan gelöst und einer Gefriertrocknung unterzogen. Das lösungsmittelfreie Gemisch der beiden Homopolymere zeigt in der flüssig-kristallinen Phase unter dem Polarisationsmikroskop eine cholesterische Grandjean-Textur.
In Analogie zu diesem Verfahren werden cholesterische Polymerphasen erhalten, wenn zu an sich bekannten nematischen Polymerphasen niedermolekulare chirale Verbindungen gemischt werden. Bevorzugt werden chirale Verbindungen gewählt, die in ihrer Molekülstruktur dem nematogenen Monomer des verwendeten nematischen Polymer ähneln.

## Patentansprüche

1. Flüssig-kristalline Polymerphase mit cholesterischer Struktur, bestehend aus mindestens einer Komponente, welche ohne Zusatz eine nematische Struktur aufweist, und mindestens einer weiteren Komponente mit chiraler Struktur, dadurch gekennzeichnet, daß sie ein Homo- oder Copolymer enthält, das nematogene Einheiten der folgenden Formel aufweist

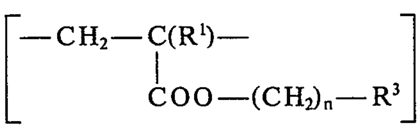

in der

$R^1$ Wasserstoff oder eine Methylgruppe ist,
n eine ganze Zahl von 1 bis 6 ist,

$R^3$ der

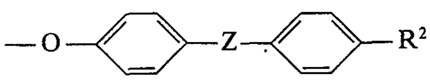

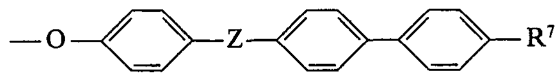

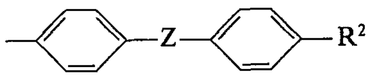

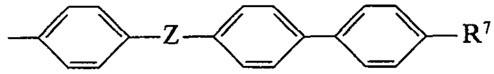

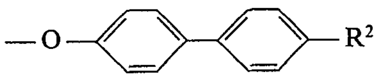

oder

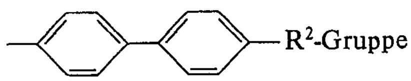

entspricht, wobei

Z eine COO- oder OCO-Gruppe ist und
$R^2$ eine $-O(CH_2)_mH$-, $-(CH_2)_mH$- oder $-COO-(CH_2)_mH$-Gruppe ist, in der m eine ganze Zahl von 1 bis 6 ist, und
$R^7$ Wasserstoff oder $R^2$ ist.

2. Polymerphase nach Anspruch 1, dadurch gekennzeichnet, daß sie ein Copolymerisat ist, welches nematogene Einheiten der Formel gemäß Anspruch 1

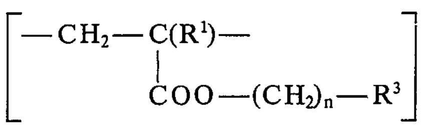

und chirale Einheiten der Formel

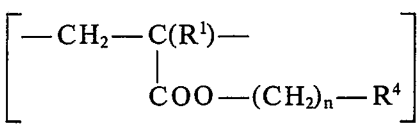

enthält, wobei in der letzteren Formel

$R^1$ Wasserstoff oder eine Methylgruppe ist,
n eine ganze Zahl von 1 bis 10 ist,
$R^4$ der

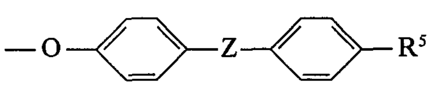

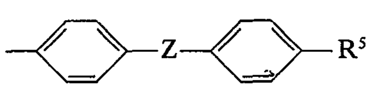

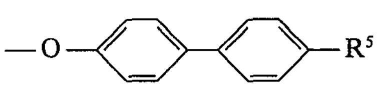

oder

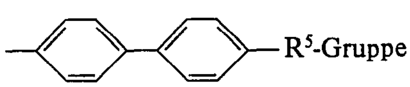

entspricht, wobei

Z eine COO- oder OCO-Gruppe ist und
$R^5$ $-CH=N-R^6$, $-OR^6$, $-COOR^6$, $-CH=CH-COOR^6$ oder $-R^6$ ist, wobei
$R^6$ eine Alkylgruppe mit 4 bis 10 C-Atomen und mindestens einem asymmetrischen C-Atom, oder den $-CH(CH_3)-C_6H_5$-Rest bedeutet.

3. Polymerphase nach Anspruch 1, dadurch gekennzeichnet, daß sie ein Gemisch aus einem Polymeren mit nematogenen Einheiten der Formel gemäß Anspruch 1

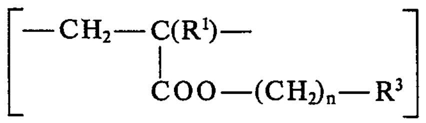

und einer niedermolekularen chiralen Verbindung und/oder einem Polymeren mit chiralen Einheiten der Formel entsprechend der zweiten Formel von Anspruch 2

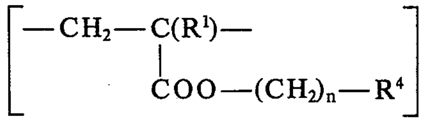

enthält.

4. Polymerphase nach einem der Ansprüche 1 bis 3, dadurch gekennzeichnet, daß $R^3$ einer

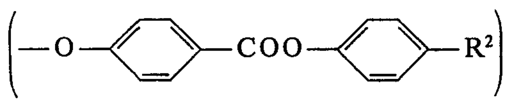 -Gruppe

entspricht.

5. Polymerphase nach einem der Ansprüche 1 bis 3, dadurch gekennzeichnet, daß $R^3$ einer

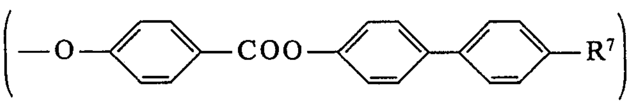 -Gruppe

entspricht.

6. Polymerphase nach einem der Ansprüche 2 oder 3, dadurch gekennzeichnet, daß $R^4$ eine

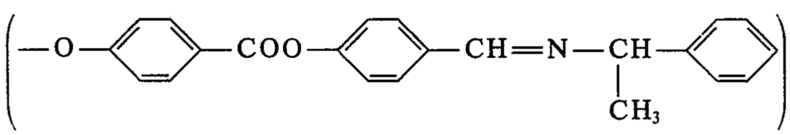 -Gruppe

ist.

7. Polymerphase nach einem der Ansprüche 2 oder 3, dadurch gekennzeichnet, daß $R^4$ eine

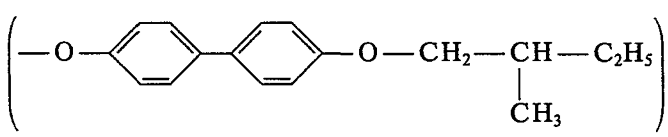 -Gruppe

ist.

8. Verfahren zur Herstellung der Polymerphase nach Anspruch 1 oder 2, dadurch gekennzeichnet, daß man Monomere der Formel

$$CH_2=C(R^1)-COO-(CH_2)_n-R^3$$

worin $R^1$, $R^3$ und n die in Anspruch 1 angegebene Bedeutung haben, und

$$CH_2=C(R^1)-COO-(CH_2)_n-R^4$$

worin $R^1$, $R^4$ und n die in Anspruch 2 für die zweite Formel angegebene Bedeutung haben, radikalisch copolymerisiert.

9. Verfahren zur Herstellung der Polymerphase nach Anspruch 1 oder 3, dadurch gekennzeichnet, daß man aus einem nematogenen Polymer mit den in Anspruch 1 angegebenen Einheiten und einer niedermolekularen chiralen Verbindung oder einem Polymeren mit chiralen Einheiten eine Mischung herstellt.

10. Verwendung der Polymerphase nach einem der Ansprüche 1 bis 9 auf dem Gebiet der Informationsspeicherung.

11. Verwendung der Polymerphase nach einem der Ansprüche 1 bis 9 als Beschichtungsmaterial.

12. Verwendung der Polymerphase nach einem der Ansprüche 1 bis 9 zur Herstellung von Polarisationsfiltern und selektiven Reflektoren.

**Claims**

1. A liquid-crystalline polymer phase having a cholesteric structure and comprising at least one component which without an additive has a nematic structure, and at least one further component having a chiral structure, which polymer phase contains a homopolymer or copolymer containing nematogenic units of the following formula

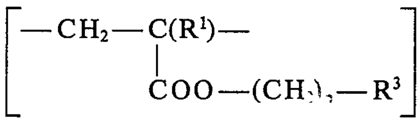

where $R^1$ is hydrogen or methyl, n is an integer from 1 to 6 and $R^3$ is

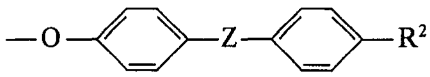

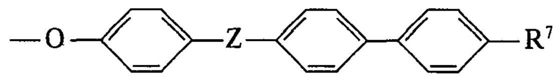

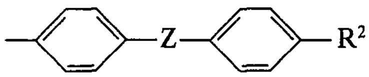

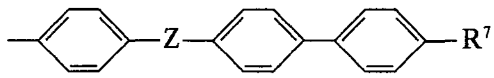

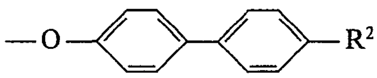

or

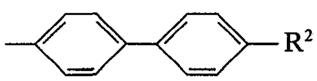

where Z is COO – or OCO –, $R^2$ is

$$—O(CH_2)_mH \quad —(CH_2)_mH \text{ or } —COO—(CH_2)_mH$$

m being an integer from 1 to 6, and $R^7$ is hydrogen or $R^2$.

2. A polymer phase as claimed in claim 1, being a copolymer which contains nematogenic units of the formula given in claim 1

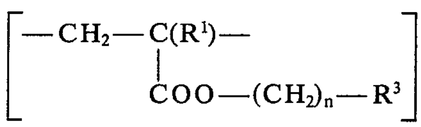

and chiral units of the formula

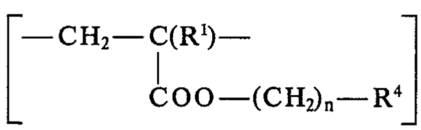

in which latter formula $R^1$ is hydrogen or methyl, n is an integer from 1 to 10 and $R^4$ is

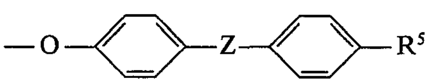

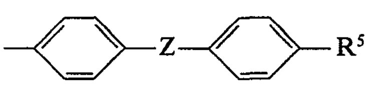

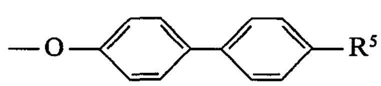

or

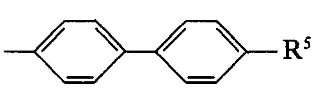

where Z is COO – or OCO – and $R^5$ is

$$—CH{=}N—R^6 \quad —OR^6 \quad —COOR^6 \quad —CH{=}CH—COOR^6 \text{ or } —R^6$$

$R^6$ being alkyl of 4 to 10 carbon atoms and having at least one asymmetric carbon atom, or $CH(CH_3)-C_6H_5$.

3. A polymer phase as claimed in claim 1, which contains a mixture of a polymer containing nematogenic units of the formula given in claim 1

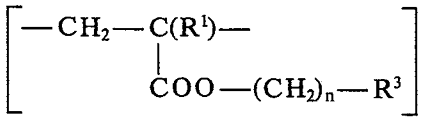

or a low molecular weight chiral compound/or a polymer containing chiral units of the second formula given in claim 2

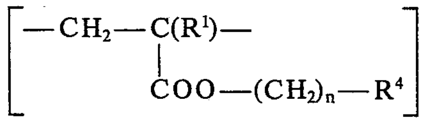

4. A polymer phase as claimed in any of claims 1 to 3, wherein $R^3$ is

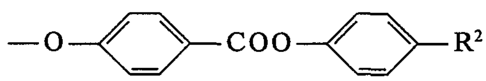

5. A polymer phase as claimed in any of claims 1 to 3, wherein $R^3$ is

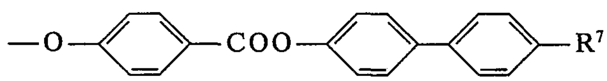

6. A polymer phase as claimed in claim 2 or 3, wherein $R^4$ is

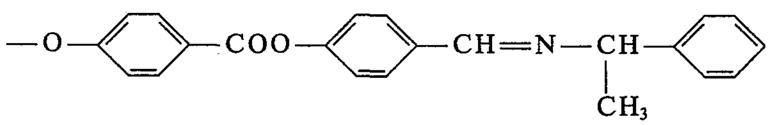

7. A polymer phase as claimed in claim 2 or 3, wherein $R^4$ is

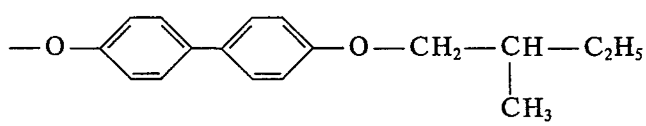

8. A process for the preparation of a polymer phase as claimed in claim 1 or 2, wherein a monomer of the formula

$$CH_2=C(R^1)COO(CH_2)_n-R^3$$

where $R^1$, $R^3$ and n have the meanings given in claim 1, and a monomer of the formula

$$CH_2=C(R^1)COO(CH_2)_n-R^4$$

where $R^1$, $R^4$ and n have the same meanings as in the second formula of claim 2, are copolymerized by a free radical mechanism.

9. A process for the preparation of a polymer phase as claimed in claim 1 or 3, wherein a mixture is prepared from a nematogenic polymer having the units given in claim 1, and a low molecular weight chiral compound or a polymer containing chiral units.

10. The use of a polymer phase as claimed in any of claims 1 to 9 in the field of information storage.

11. The use of a polymer phase as claimed in any of claims 1 to 9 as a coating material.

12. The use of a polymer phase as claimed in any of claims 1 to 9 for the production of polarization filters and selective reflectors.

**Revendications**

1. Phase polymère liquide-cristalline à structure cholestérique, constituée d'au moins une composante possédant sans addition une structure nématique et d'au moins une autre composante à structure chirale, caractérisée en ce qu'elle contient un homopolymère ou un copolymère comprenant des unités nématogènes de la formule

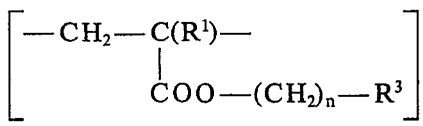

dans laquelle

$R^1$ désigne un atome d'hydrogène ou un radical méthyle,
n est un nombre entier valant 1 à 6 et
$R^3$ représente un groupe de l'une des formules

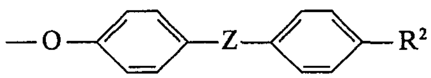

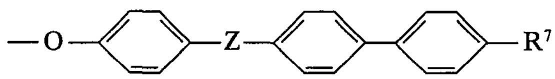

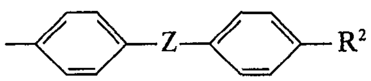

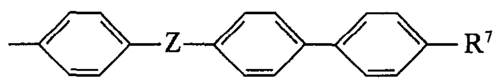

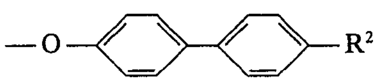

et

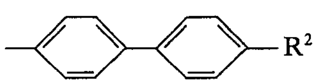

où

Z désigne un groupe COO– ou OCO– et
$R^2$ représente un groupe $-O(CH_2)_mH$, $-(CH_2)_mH$ ou $-COO-(CH_2)_mH$,
m est un nombre entier valant 1 à 6 et
$R^7$ désigne un atome d'hydrogène ou possède l'une des significations de $R^2$.

2. Phase polymère suivant la revendication 1, caractérisée en ce qu'elle est constituée d'un copolymérisat comprenant des unités nématogènes de la formule

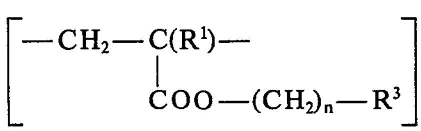

selon la revendication 1 et des unités chirales de la formule

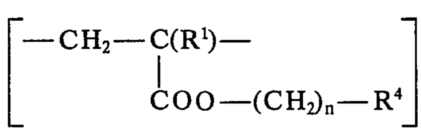

dans laquelle

$R^1$ désigne un atome d'hydrogène ou un radical méthyle,
n est un nombre entier valant 1 à 10 et
$R^4$ représente un groupe de l'une des formules

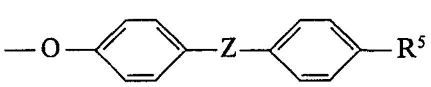

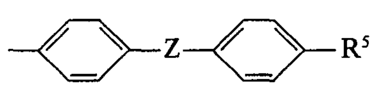

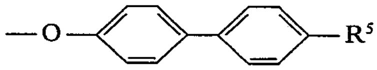

et

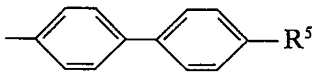

où

Z désigne un groupe COO – ou OCO – et
$R^5$ représente un groupe $-CH=N-R^6$, $-OR^6$, $-COOR^6$, $-CH=CH-COOR^6$ ou $-R^6$,
$R^6$ représentant un radical alcoyle en $C_4$ à $C_{10}$ avec au moins un atome de carbone asymétrique ou le groupe $-CH(CH_3)-C_6H_5$.

3. Phase polymère suivant la revendication 1, caractérisée en ce qu'elle est constituée d'un mélange d'un polymère à unités nématogènes de la formule

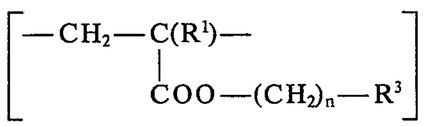

selon la revendication 1 et d'un composé chiral à bas poids moléculaire et(ou) d'un polymère à unités chirales de la formule

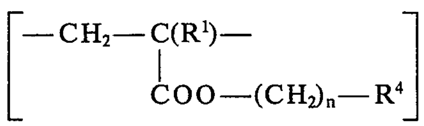

selon la revendication 2.

4. Phase polymère suivant l'une des revendications 1 à 3, caractérisée en ce que $R^3$ représente un groupe

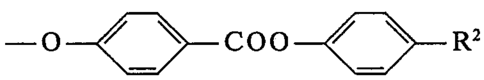

5. Phase polymère suivant l'une des revendications 1 à 3, caractérisée en ce que $R^3$ représente un groupe

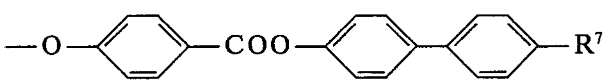

6. Phase polymère suivant l'une des revendications 2 et 3, caractérisée en ce que $R^4$ représente un groupe

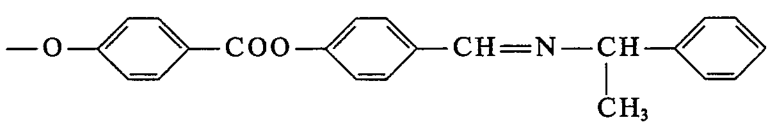

7. Phase polymère suivant l'une des revendications 2 et 3, caractérisée en ce que $R^4$ représente un groupe

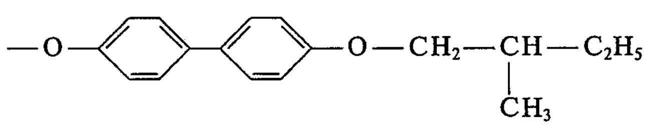

8. Procédé de préparation d'une phase polymère suivant la revendication 1 ou 2, caractérisé en ce que l'on soumet à une copolymérisation radicalaire des monomères de la formule

$$CH_2=C(R^1)-COO-(CH_2)_n-R^3$$

dans laquelle $R^1$, $R^3$ et n possèdent la signification définie dans la revendication 1, et des monomères de la formule

$$CH_2{=}C(R^1)-COO-(CH_2)_n-R^4$$

dans laquelle $R^1$, $R^4$ et n possèdent la signification définie pour la deuxième formule de la revendication 2.

9. Procédé de préparation d'une phase polymère suivant la revendication 1 ou 3, caractérisé en ce que l'on prépare un mélange d'un polymère nématogène comprenant des unités définies dans la revendication 1 et d'un composé chiral à bas poids moléculaire ou d'un polymère à unités chirales.

10. Utilisation d'une phase polymère suivant l'une des revendications 1 à 9 dans le domaine de l'enregistrement de données.

11. Utilisation d'une phase polymère suivant l'une des revendications 1 à 9 comme matière d'enduction.

12. Utilisation d'une phase polymère suivant l'une des revendications 1 à 9 pour la fabrication de filtres de polarisation et de réflecteurs sélectifs.